# NEW EUROPEAN PATENT SPECIFICATION

(11) **EP 0 977 883 B2**
(45) Date of publication and mention of the opposition decision: **01.08.2007**
(45) Mention of the grant of the patent: 06.10.2004
(21) Application number: 98921489.5
(22) Date of filing: 22.04.1998
(51) Int. Cl.: C12P 37/06

(54) **IMPROVED PROCESS FOR THE FERMENTATIVE PRODUCTION OF PENICILLIN**
VERBESSERTES VERFAHREN ZUR FERMENTATIVER HERSTELLUNG VON PENIZILLIN
PROCEDE AMELIORE DE PRODUCTION FERMENTATIVE DE PENICILLINE

(30) Priority: 22.04.1997 EP 97201200
(43) Date of publication of application: 09.02.2000
(60) Divisional application: 04103240.0
(73) Proprietor: DSM IP Assets B.V., 6411 TE Heerlen (NL)
(72) Inventor: KERKHOF, Pieter, Theodorus, NL-3191 HS Hoogvliet (NL); KUIPERS, Rienk, Hendrik, NL-2804 KZ Gouda (NL); WALRAVEN, Hubertus, Gerardus, Maria, NL-2284 TM Rijswijk (NL)
(74) Representative: Elkenbracht, Johan Christiaan
(86) International application number: PCT/EP1998/002462
(87) International publication number: WO 1998/048039

(56) References cited:
- EP-A- 0 122 681
- WO-A-82/01563
- WO-A-97/35029
- DE-A- 3 729 338
- DE-A- 4 005 366
- FR-A- 1 299 883

## Description

### FIELD AND BACKGROUND OF THE INVENTION

The invention relates to a process for the preparation of penicillins, which are deacylated at the 6-amino group, by the fermentation of a penicillin producing microorganism in the presence of a side chain precursor and by deacylation of fermentatively produced N-substituted penicillin.

6-Amino penicillanic acid (6-APA) is an important intermediate in the pharmaceutical industry which is in general obtained through chemical or enzymatic cleavage ('deacylation') of penicillin G or penicillin V.

Penicillins themselves are obtained from fermentation using strains of *Penicillium chrysogenum*. In a typical penicillin G recovery process, the broth is filtered and washed, and penicillin G is extracted to butyl acetate or methyl isobutyl ketone after acidification of the filtrate. The solvent is then decolorized with aid of active carbon and the penicillin G acid is back-extracted to water upon neutralization with an aqueous potassium salt solution such as potassium acetate. This solution is admixed with sufficient butanol, the water content is reduced using evaporation of the butanol-water azeotrope, whereupon the penicillin G crystals formed are recovered by filtration and subsequent washing and drying.

In another process the crystalline material is directly obtained from the organic solution by addition of potassium acetate or other potassium salts and evaporation of the azeotrope, whereupon the crystals are filtered, washed with butanol, filtered and dried. For penicillin V the same process can be applied. However, due to better stability of penicillin V under acidic conditions, penicillin V can also be crystallized in the acid form instead of the potassium salt form, without economical unacceptable losses.

6-Amino penicillanic acid is in general obtained from a solution of dissolved solid penicillin G or V, which is then contacted with immobilized penicillin G acylase or penicillin V acylase, respectively. The conversion solution is subsequently fed to a crystallizer where the solid product is formed by precipitation at a pH of about 3.5 to 4.5. Generally, in this crystallization process the pH is lowered stepwise to avoid contamination of the final product 6-APA with high side chain levels and coloured compounds. In addition, solvents like methanol, ethanol, iso propyl alcohol, are added to avoid this unacceptable contamination. It will be clear that these types of production routes to 6-APA are lengthy and costly.

The present invention discloses an improved method for the production of 6-APA, wherein the isolation of penicillin G or V as a solid intermediate is not required and wherein the colour correction measures are made redundant.

### DESCRIPTION OF THE INVENTION

The present invention discloses an improved process for the production of 6-amino penicillanic acid (6-APA) from a fermentation broth of a penicillin producing microorganism.

In general the novel production and purification process comprises the following steps:

A penicillin-producing microorganism is fermented in the presence of a suitable side chain precursor. The fermentation broth is filtered, whereupon the filtrate is extracted with an organic solvent to obtain an organic phase containing the major part of N-substituted penicillanic acid. Subsequently, the N-substituted penicillanic acid is back extracted to water. The aqueous phase optionally is stripped to remove traces of solvent. The remaining aqueous solution phase is enzymatically treated to yield 6-APA and a side chain, as for instance phenoxy or phenyl acetic acid.

Subsequently, 6-APA is precipitated from the aqueous conversion solution after acidification of said solution and isolated as a crystalline product. The steps of extraction, back extraction and crystallization are carried out in a continuous mode. However, a more preferred embodiment of the invention comprises acidification of the conversion solution and subsequent extraction of the side chain to an organic solvent prior to precipitation of 6-APA from the aqueous phase using a pH-shift.

In the process according to the invention, the following steps are described in more detail.

A fermentation broth containing the N-substituted penicillin is obtained from any suitable fermentation process, e.g from a fermentation of a strain of *Penicillium chrysogenum* in the presence of a suitable side chain precursor, such as phenyl acetic acid or phenoxy acetic acid.

The biomass is separated from the fermentation broth using any suitable technology, such as centrifugation or filtration, yielding a penicillin-containing fermentation fluid. Preferably, a filtration step is applied to obtain said separation. The residual solids optionally are washed.

The fermentation broth or fluid is acidified to a pH within a range of 2-4, preferably to a pH within a range of 2.5-3, by addition of an acid, such as sulfuric acid, hydrochloric acid or nitric acid or a combination thereof. The N-substituted penicillin is then separated from the acidified aqueous phase by extraction to an organic solvent. The organic solvent which is used preferably is amyl acetate, butyl acetate, ethyl acetate, methyl isobutyl ketone, cyclohexanone, iso-butanol or n-butanol. The addition of a suitable de-emulsifier may improve the extraction significantly.

In one embodiment of the invention, part of the organic solvent may be added to the fermentation broth or fluid prior to acidification. This was found to be satisfactory in avoiding precipitation, said precipitation sometimes occurring as an undesired side effect of acidification of the fermentation broth or fluid. Precipitation is undesired since it will lead to loss of product and may cause subsequent operational problems.

In a next step, the N-substituted penicillin as present in the organic solvent is back extracted to water at a pH of 6-10, preferably at a pH of 7-9. The base used for pH adjustment for instance can be ammonia or an alkaline or earth alkaline hydroxide solution.

After phase separation, the aqueous phase optionally is stripped to remove the organic solvent.

Subsequently, the aqueous phase or solution is treated with a suitable penicillin acylase.

Organisms that have been found to produce penicillin acylase are, for example, *Acetobacter, Aeromonas, Alcaligenes, Aphanocladium, Bacillus* sp., *Cephalosporium, Escherichia, Flavobacterium, Kluyvera, Mycoplana, Protaminobacter, Providentia, Pseudomonas* or *Xanthomonas* species. Enzymes derived from *Acetobacter pasteurioanum, Alcaligenes faecalis, Bacillus megaterium, Escherichia coli, Providentia rettgeri* and *Xanthomonas citrii* have particularly proven to be succesful in a method according to the invention. In the literature, penicillin acylases have also been referred to as penicillin amidases.

The penicillin acylase may be used as a free enzyme, but also in any suitable immobilized form, for instance as has been described in EP 0 222 462 and WO 97/04086.

It is advantageously shown by the present invention that is is feasible to subject a fermentatively derived N-substituted penicillin compound, such as penicillin G, to an enzymatic conversion to 6-APA without the necessity for prior isolation of the N-substituted penicillin compound in a crystalline form.

The conversion solution, resulting from the enzymatic reaction indicated above, contains 6-APA, a side chain such as phenyl or phenoxy acetic acid, coloured products and traces of unconverted product.

In one embodiment of the invention, the product 6-APA is isolated from the conversion solution by crystallization at acidic conditions. Typically, crystallization of 6-APA from an aqueous 6-APA solution is performed by adjusting the pH of the 6-APA solution to an acidic value by adding a titrant to the 6-APA solution until the pH has reached a value within a range of 2.5-4.5, preferably a value of 3-4.

In a preferred embodiment of the invention, crystallization of 6-APA from an aqueous solution is carried out by adding the 6-APA solution to a crystallization vessel which is kept at a fixed pH having a value within a range of 2.5-4.5, using a suitable titrant.

In an even more preferred embodiment of the invention, said crystallization is carried out by a stepwise adjustment of the pH of the 6-APA-solution to a final value within a range of 2.5-4.5 by adding the 6-APA solution to a series of interconnected crystallization vessels, i.e. adding the 6-APA solution to a first vessel, simultaneously adding the content of the first vessel to a second vessel, simultaneously adding the content of the second vessel to a third vessel, etc., wherein a pH range is applied in the interconnected vessels using a suitable titrant, starting at a pH in the first vessel which deviates about 0.5-2 pH units from the pH of the 6-APA solution and ending at a pH in the final vessel which has a value within a range of 2.5-4.5. Conveniently, the pH of the aqueous 6-APA solution is adjusted to the desired final value using a series of 2-6 interconnected vessels.

For instance, to obtain crystallization of 6-APA from the conversion solution, a decreasing pH range from 8 to 3 can be applied using a titrant which is an acid, such as sulfuric acid, hydrochloric acid and/or nitric acid, applying a series of 3-4 interconnected crystallization vessels.

The two preferred embodiments described above are performed in a continuous mode.

In a further preferred embodiment of the invention, the phenyl or phenoxy acetic acid side chain is removed prior to crystallization of 6-APA by extraction to an organic solvent. The conversion solution is acidified and then extracted with an organic solvent, for instance amyl acetate, butyl acetate, ethyl acetate, methyl isobutyl ketone, cyclohexanone, iso-butanol or n-butanol. The acidification of the conversion solution is performed with an acid, such as sulfuric acid, hydrochloric acid or nitric acid or a combination thereof, preferably sulfuric acid, to a pH lower than 3, preferably lower than 2. It was unexpectedly found that also a high removal efficiency of the coloured impurities was obtained upon applying this extraction step.

In one embodiment of the invention, the organic solvent extraction is performed in two to four stages of intensive contact extraction, for example a combination of an intense mixer, for instance a high shear mixer with a centrifugal separation, said stages preferably operated in counter current mode.

The organic solvent, containing phenyl or phenoxy acetic acid and coloured compounds, may contain a small quantity of 6-APA. Said 6-APA may optionally be back extracted to water, preferably in two to four extraction stages, in counter current mode. The water used for this back extraction is acidified to a pH lower than 6, preferably lower than 3. The preferred water:solvent ratio is 1:5, more preferably 1:20, most preferably 1:10. The thus-obtained aqueous phase can be fed to the 6-APA crystallization or recycled into the side chain extraction.

After organic solvent extraction of the side chain, the 6-APA product can be crystallized from the aqueous phase in several ways, such as the ways which are indicated hereinabove for crystallization of 6-APA from an aqueous solution.

In a preferred mode of operation, the pH of the aqueous phase is increased to a pH having a value within a range of 2.5-5, preferably within a range of 3.5-4.5, by adding the 6-APA solution in one step to a crystallization vessel kept at the desired pH value or to a series of 2-6 interconnected crystallization vessels applying an increasing pH range. These processes are carried out in continuous mode.

When applying this aspect of the process of the invention, the amount of side chain, e.g. phenyl or phenoxy acetic acid, and coloured compounds present in the 6-APA crystals is low as compared to conventional processes wherein the side chain is not extracted and batch mode crystallization from a neutral solution is applied.

All of the above mentioned steps, i.e. extraction, back extraction and crystallization, are carried out, because of stability reasons in a continuous mode.

The obtained 6-APA crystals are isolated by filtration or centrifugation and dried in a conventional continuous or batch dryer.

## Claims

1. Process for the preparation of 6-amino penicillanic acid (6-APA) from a fermentatively produced N-substituted penicillin, comprising the steps of:
extraction of the N-substituted penicillin compound as present in a fermentation broth or fluid to an organic solvent, back extraction of the N-substituted penicillin compound to water, treatment of the aqueous phase with a penicillin acylase and isolation of the 6-APA from the thus-obtained conversion solution by crystallization, **characterized in that** the steps of extraction, back extraction and crystallization are carried out in a continuous mode.

2. Process according to claim 1, further **characterized in that** the side chain as present in the conversion solution obtained after acylase treatment is extracted to an organic solvent prior to isolation of 6-APA from the aqueous phase by crystallization.

3. Process according to claim 2, wherein the conversion solution is acidified to a pH lower than 3 prior to extraction.

4. Process according to any one of the claims 1 to 3, wherein crystallization of 6-APA from the conversion solution or aqueous phase is performed by adding the 6-APA solution to a crystallization vessel which is kept at a fixed pH having a value within a range of 2.5-4.5, using a suitable titrant.

5. Process according to any one of the claims 1 to 3, wherein crystallization of 6-APA from the conversion solution or aqueous phase is performed by adding the 6-APA solution to a series of interconnected crystallization vessels while applying a pH range using a suitable titrant, starting at a pH in the first vessel which deviates about 0.5-2 pH units from the pH of the 6-APA solution and ending at a pH in the final vessel which has a value within a range of 2.5-4.5.

6. Process according to any one of the claims 1 to 5, wherein the fermentation broth or fluid is acidified to a pH within a range of 2-4, preferably within a range of 2.5-3, prior to extraction.

7. Process according to claim 6, wherein part of the organic solvent is added to the fermentation broth or fluid prior to acidifying said broth or fluid.

8. Process according to any one of the claims 1 to 7, wherein the aqueous phase is stripped prior to treatment with a penicillin acylase.

9. Process according to any one of the claims 1 to 8, wherein the organic solvent is amyl acetate, butyl acetate, ethyl acetate, methyl isobutyl ketone, cyclohexanone, iso-butanol or n-butanol.

## Patentansprüche

1. Verfahren zum Herstellen von 6-Aminopenicillansäure (6-APA) aus einem durch Fermentation hergestellten N-substituierten Penicillin, mit folgenden Stufen:
Extraktion des in einer Fermentationsbrühe oder einem Fermentationsfluid vorliegenden N-substituierten Penicillin in ein organisches Lösungsmittel, Rück-extraktion des N-substituierten Penicillins in Wasser, Behandlung der wässrigen Phase mit einer Penicillin-acylase und Isolierung der 6-APA aus der so erhaltenen Umwandlungslösung durch Kristallisation, **dadurch gekennzeichnet daß** die Stufen Extraktion, Rück-extraktion und Kristallisation werden ausgeführt in einem kontinuierliche Weise.

2. Verfahren nach Anspruch 1, ferner **dadurch gekennzeichnet, daß** die Seitenkette, die in der nach der Acylasebehandlung erhaltenen Umwandlungslösung vorliegt, in ein organisches Lösungsmittel extrahiert wird, bevor die 6-APA aus der wässrigen Phase durch Kristallisieren isoliert wird.

3. Verfahren nach Anspruch 2, worin vor der Extraktion die Umwandlungslösung auf einen pH-Wert unter 3 angesäuert wird.

4. Verfahren nach einem der Ansprüche 1 bis 3, worin die Kristallisation von 6-APA aus der Umwandlungslösung oder der wässrigen Phase durch Zuführen der 6-APA-Lösung in einen Kristallisationsbehälter durchgeführt wird, der unter Einsatz einer geeigneten Titrier-standardlösung auf einem festen pH-Wert im Bereich von 2,5 bis 4,5 gehalten wird.

5. Verfahren nach einem der Ansprüche 1 bis 3, worin die Kristallisation von 6-APA aus der Umwandlungslösung oder der wässrigen Phase durch Zugabe der 6-APA-Lösung zu einer Reihe von miteinander verbundenen Kristallisationsbehältern durchgeführt wird, wobei ein pH-Bereich unter Einsatz einer geeigneten Titrierstandardlösung eingestellt wird sowie in dem ersten Behälter bei einem pH-Wert begonnen wird, der um etwa 0,5 bis 2 pH-Einheiten vom pH-Wert der 6-APA-Lösung abweicht, und das Ende in dem letzten Behälter bei einem pH-Wert erreicht wird, der im Bereich von 2,5 bis 4,5 liegt.

6. Verfahren nach einem der Ansprüche 1 bis 5, worin vor der Extraktion die Fermentationsbrühe oder das Fermentationsfluid auf einen pH-Wert in einem Bereich von 2 bis 4, vorzugsweise von 2,5 bis 3, angesäuert wird.

7. Verfahren nach Anspruch 6, worin ein Teil des organischen Lösungsmittels der Fermentationsbrühe oder dem Fermentationsfluid zugegeben wird, bevor diese Brühe oder dieses Fluid angesäuert wird.

8. Verfahren nach einem der Ansprüche 1 bis 7, worin die wässrige Phase vor dem Behandeln mit einer Penicillinacylase abgezogen wird.

9. Verfahren nach einem der Ansprüche 1 bis 8, worin das organische Lösungsmittel Amylacetat, Butylacetat, Ethylacetat, Methylisobutylketon, Cyclohexanon, Iso-butanol oder n-Butanol ist.

## Revendications

1. Procédé de préparation d'acide 6-aminopénicillinique (6-APA) à partir d'une pénicilline N-substituée produite par fermentation, incluant les étapes suivantes :
extraction du composé de pénicilline N-substituée, présent dans un bouillon ou fluide de fermentation, pour le transférer dans un solvant organique ; réextraction du composé de pénicilline N-substituée pour le transférer dans de l'eau ; traitement de la phase aqueuse à l'aide d'une acylase de pénicilline et isolation, par cristallisation, du 6-APA vis-à-vis de la solution de conversion ainsi obtenue, **caractérisé en que** les étapes d'extraction, réextraction et cristallisation sont executé dans une façon continue.

2. Procédé selon la revendication 1, **caractérisé en outre en ce que** la chaîne latérale, présente dans la solution de conversion obtenue après le traitement par l'acylase est extraite pour être transférée dans un solvant organique avant l'isolation, par cristallisation, du 6-APA vis-à-vis de la phase aqueuse.

3. Procédé selon la revendication 1 ou 2, dans lequel la solution de conversion est acidifiée de manière à obtenir un pH inférieur à 3 avant l'extraction.

4. Procédé selon l'une quelconque des revendications 1 à 3, dans lequel la cristallisation du 6-APA à partir de la solution de conversion ou de la phase aqueuse est effectuée en versant la solution de 6-APA dans une cuve de cristallisation maintenue, à l'aide d'une solution titrée appropriée, à un pH fixé dont la valeur se situe entre 2,5 et 4,5.

5. Procédé selon l'une quelconque des revendications 1 à 3, dans lequel la cristallisation du 6-APA à partir de la solution de conversion ou de la phase aqueuse est effectuée en versant la solution de 6-APA dans une série de cuves de cristallisation interconnectées tout en faisant intervenir, à l'aide d'une solution titrée appropriée, une certaine gamme de pH : à commencer par le pH dans la première cuve qui diffère d'environ 0,5 à 2 unités du pH de la solution de 6-APA, pour finir par le pH dans la dernière cuve dont la valeur se situe entre 2,5 et 4,5.

6. Procédé selon l'une quelconque des revendications 1 à 5, dans lequel le bouillon ou fluide de fermentation est acidifié de manière à obtenir un pH compris entre 2 et 4, et de préférence entre 2,5 et 3, avant l'extraction.

7. Procédé selon la revendication 6, dans lequel une partie du solvant organique est ajoutée au bouillon ou fluide de fermentation avant l'acidification dudit bouillon ou fluide.

8. Procédé selon l'une quelconque des revendications 1 à 7, dans lequel la phase aqueuse est ôtée avant le traitement par une acylase de pénicilline.

9. Procédé selon l'une quelconque des revendications 1 à 8, dans lequel le solvant organique est l'acétate d'amyle, l'acétate de butyle, l'acétate d'éthyle, la méthylisobutylcétone, la cyclohexanone, l'isobutanol ou le n-butanol.
